# EUROPEAN PATENT SPECIFICATION

(11) **EP 3 445 255 B1**
(45) Date of publication and mention of the grant of the patent: **08.04.2020**
(21) Application number: 16735947.0
(22) Date of filing: 22.04.2016
(51) Int. Cl.: A61B 17/06

(54) **MODULAR SURGICAL WIRE FOR PLASTIC SURGERY, DERMATOLOGICAL SURGERY, COSMETIC SURGERY**
MODULARER CHIRURGISCHER DRAHT FÜR PLASTISCHE CHIRURGIE, DERMATOLOGISCHE CHIRURGIE, KOSMETISCHE CHIRURGIE
FIL CHIRURGICAL MODULAIRE POUR CHIRURGIE PLASTIQUE, CHIRURGIE DERMATOLOGIQUE, CHIRURGIE ESTHÉTIQUE

(43) Date of publication of application: 27.02.2019
(73) Proprietor: PromoItalia Group S.p.A., 20124 Milano (MI) (IT)
(72) Inventor: MATANO', Valerio, 80122 - Napoli (NA) (IT); TRANFAGLIA, Egidio, 80122 Napoli (NA) (IT); ACCARDO, Ciro, 81050 Portico di Caserta (CE) (IT)
(74) Representative: Sarpi, Maurizio
(86) International application number: PCT/IT2016/000100
(87) International publication number: WO 2017/183049

(56) References cited:
- EP-A1- 2 108 316
- EP-A1- 2 446 832

## Description

### FIELD OF THE INVENTION

The present invention relates to the sector of aesthetic medicine, and to the materials for carrying out operations of general and specialized surgery, of plastic, dermatological, and aesthetic surgery, of gynaecological and/or obstetric surgery, as well as for rhinoplasty and urological and proctological surgery.

More specifically, the invention regards the use of biomedical and medical materials, such as absorbable and/or permanent threads that are used as fillers or as sutures for tissue lifting, as repositioning sutures or as retention sutures or sutures for aggregation or compaction or for joining edges of skin, subcutaneous tissue, muscles, tendons, and/or connective fasciae, etc. Threads or sutures of this type are moreover used for mini-invasive perineorraphy and/or osteoplasty and/or mini-invasive vaginal osteorraphy and/or for vaginal tightening and/or for anal tightening (for treatment of faecal incontinence).

In this particular field, the subject of the invention is an absorbable and/or permanent monofilament or multifilament surgical thread in which the single strand has the peculiar characteristic of being modular, i.e., of being constituted by a plurality of moulded basic units or elements, made of biosensitive material, such as, but not exclusively, a plastic polymer, which have a well-defined shape and size and can be assembled together.

Also forming the subject of the invention is a method for producing the aforesaid thread, which envisages assembling together the individual modular units (which can be obtained with a traditional ultrafine moulding technology or resorting to more sophisticated technologies, such as micro-technology and nano-technology) in a pre-ordered sequence that the suture being produced must present, and performing a subsequent thermal treatment in a welding oven, where the individual basic units, once they are duly assembled together, are stably welded to one another following upon a well-defined temperature gradient that depends upon the material used.

The specific material of which the individual modular units are obtained by moulding (preferably, but not exclusively, a thermoplastic polymer) responds to the specific temperature gradient with a shrinkage in its original volume such as to render disassembly impossible. Surgical threads of the desired length are thus obtained, present in which are sequences of barbs or protuberances, which may also have different orientations, maintaining the gauge regular throughout the length of the thread, whether it is a fixed gauge or an increasing or decreasing gauge. This result constitutes an absolute novelty since the sutures currently being produced are mostly obtained by extrusion, with the inevitable drawback, of the manifest and undesirable variability of their thickness or gauge.

The above variability has led to identification, in the USA, for the gauge of sutures, of ranges of acceptable values with respect to the mean value. These data are provided in tables drawn up by the United States Pharmacopoeia (USP).

### PRIOR ART

In the current state of the art, free multifilament sutures, or free monofilament sutures, to be used for the surgical operations already mentioned previously, are provided with barbs or protuberances, which may be distributed with convergent and/or divergent bidirectional geometry or with unidirectional geometry or with a stochastic arrangement.

The barbs or protuberances, which may be present throughout the length of the thread, present the peculiarity of hindering sliding or displacement (with respect to the major axis of the thread), the so-called shift, in the two opposite directions when the thread is inserted into human soft tissue, or into tendons, or into fasciae, or into the periosteum, or into the perichondrium, or into muscular tissue, or also for suturing the hollow viscera of the human body.

Shifting, according to the major axis of the thread, is produced by the tensile forces exerted both on throughout the length of the suture, by transverse forces (due to the dynamic action of the striated and/or smooth muscle of the anatomical district in which it is placed), and on both ends of the thread. These tensile forces occur as a result of dynamic and mechanical actions due to the activity of voluntary or involuntary muscles, for example, during facial mimicry, mastication, ambulation, physiological activities typical of micturition, sexual activity, defecation, the actions performed by the smooth muscle of the alimentary canal, etc.

The sutures in question are referred to as "free" because they do not possess anchoring points either upstream or downstream throughout their development. It to be recalled that if the monofilament suture is referred to as "convergent bidirectional suture", the apex of each barb is directed or oriented towards the mid point of the free suture, if the suture is referred to as "divergent bidirectional suture", the apex of each barb is directed or oriented towards the closer end of the free suture, and finally, if the suture is referred to as "unidirectional suture", the apices of the barbs are oriented in one and the same direction for all the barbs.

Numerous manufacturers are holders of patents for design of sutures. Sutures provided with barbs or protrusions with characteristic arrangements and shapes are widely described in numerous patent documents (Alcamo, US patent 3123077; Akiyama, US patent 4069825, Fukuda, US patent 4467805; Ruff, US patent 5.342.376; Promoitalia International, patent WO06/061868; EP-A-2446832; EP-A-2108316).

A simple analysis leads us to the observation that the types of barbed sutures available on the market are basically characterized as regards:
direction - the barbs may be in fact be arranged in one and the same direction (unidirectional sutures) or in two directions (bidirectional sutures); in the case of bidirectional barbs, once the sutures are inserted into a hollow needle and properly positioned, they are unable to move on account of the bidirectionality of the fixing barbs, thus guaranteeing a greater stability; and
side: the barbs may be arranged either on just one side of the thread (unilateral sutures) or on both sides of the thread (bilateral sutures); currently, threads with spiral arrangement of the barbs (3D sutures) are also widely used, which cover the entire surface of the thread, guaranteeing a higher tension.

There are not, however, available on the market threads that have in succession, along their length, sequences of barbs with different characteristics, which are desirable in some situations where, for example, stretches without barbs are followed by stretches with bilateral opposed barbs and/or stretches of barbs arranged in a spiral or divergent stretches, and this owing to the difficulties that, with the techniques available, are currently encountered in providing on one and the same thread barbs that are differently oriented and arranged.

There is consequently felt the considerable need to provide innovative surgical sutures that will enable the above requirements to be met, which arise above all in certain sectors of plastic, general, and specialized surgery.

### OBJECT OF THE INVENTION

The invention is defined in claim 1. Certain optional features of the invention are defined in the dependent claims. The object of the present invention is a new technologically advanced type of suture, which may be made no longer like traditional biodegradable absorbable permanent sutures, which resort to industrial technologies of extrusion and/or moulding, but by assembling together basic micro-units of well-defined shape and size, made of thermosensitive material, which are obtained with a conventional technique of casting in a mould, or with other technologies currently available such as microtechnologies and nano-technologies, and present characteristics of modularity since they are provided with coupling means co-operating together that enable mutual coupling in the production stage, like elements of the game Lego, to obtain both smooth sutures and sutures with protuberances or barbs or protrusions, or different forms according to the different multiple modes of assembly or slotting together of the specific basic units, or also sutures with a diameter that increases or decreases in the direction of their length.

An object of the present disclosure is also the method for producing a thread having the desired characteristics and the desired length starting from said micro-units, which envisages the steps of:
1) assembling together the micro-units having the desired characteristics, thanks to the coupling means with which they are equipped, which co-operate with one another; and
2) placing the sutures thus assembled in a heating oven at controlled temperatures in order to weld them stably together and thus obtain a thread having the desired characteristics and the desired length by subjecting them to specific temperature gradients (ΔT), with repeated and alternating heating and cooling cycles, where the range of the temperature gradient that is used depends upon the specific material used.

By way of indication, the above temperature gradient, using, according to a preferred embodiment, the biodegradable polymers referred to as "lactones", falls in the range between Tg (glass-transition temperature) and Tm (melting point), i.e., a temperature gradient comprised between -20°C and + 400°C.

By "glass-transition temperature" is meant the phase transition whereby an amorphous polymeric material (or else the part with amorphous structure of a polymeric material that has a partially crystalline structure) changes from a plastic state into a hard and typically brittle state. This transition occurs in a narrow range around a characteristic temperature, referred to as "glass-transition temperature".

The glass-transition values of common reference are in actual fact mean values, this quantity depending upon the temperature gradient with which cooling is carried out and, for polymers, also upon the distribution of the mean molecular weights. Moreover, the possible presence of additives may also affect Tg.

It is known by a person skilled in the branch that, if a polymer has a Tg higher than the desired one, it is sufficient to add a so-called plasticizer to the polymer (such as the plasticizer TEHTM, [tris(2-ethylhexyl)benzene-1,2,4-tri-carboxylate]). This is a small molecule that positions itself between the polymeric chains and spaces them apart from one another. This process is referred to as "increase in the free volume". When this occurs, the chains can slide closer to one another more easily. When they slide closer to one another more easily, they can move freely at lower temperatures than in the case of absence of plasticizer. In this way, the Tg of a polymer can be reduced in order to render a polymer more flexible and easier to work.

When the polymer is cooled below this temperature, it becomes hard and brittle like glass. Some polymers are used above their glass-transition temperatures, and some below. To a first approximation, below their Tg they behave as solid materials, whereas above their Tg they behave as rubbery or flexible materials, like sutures.

Melting occurs in a crystalline polymer, whereas glass transition occurs only in polymers in the amorphous state. A given polymer will always have both the amorphous part and the crystalline part inside it so that one and the same sample can always have a Tm and a Tg. The chains that melt are not the same chains as those that undergo glass transition.

In a preferred embodiment, it is envisaged that assemblage is performed by means of a robotized industrial machine that is able to handle said basic units, which are of dimensions of the order of even one hundredth of a millimetre.

Further characteristics and advantages of the invention will emerge clearly from the ensuing detailed description with reference to the attached plates of drawings, which illustrate purely by way of nonlimiting example some preferred embodiments of the basic units of the modular suture.

### BRIEF DESCRIPTION OF THE DRAWINGS

In the attached plates of drawings:
Figure 1 is a schematic elevation of a modular element constituted by a basic unit without barbs provided with a female coupling means set at one of its free ends and with a male coupling means set at the other free end, which enable assembly thereof with other N basic units provided with similar coupling means so as to give rise to a portion without barbs, having the desired length, of a linear final suture of greater length;
Figure 2 is a perspective view from above of the same basic unit;
Figure 3 is a perspective view from beneath of the same basic unit;
Figure 4 shows in vertical section a female-female basic unit;
Figure 5 shows in vertical section a male-male basic unit;
Figure 6 shows in vertical section a female-male basic unit, with the real parameterized dimensions that reproduce all the USP technical specifications;
Figures 7 and 8 are, respectively, a top plan view (female side) of the female-male basic unit and a plan view from beneath (male side) of the same basic unit;
Figure 9 is a perspective view of a basic unit provided with a single barb that, once assembled with other similar ones, gives rise to a portion with barbs of a suture of greater length, which may be a unidirectional suture or a divergent or convergent bidirectional suture or a suture where said portion alternates with other barbed stretches;
Figure 10 shows a basic unit with a single barb in a position specular to that of Figure 9 to enable production of sutures with alternately opposed barbs, either convergent or divergent, and with a linear or spiral development;
Figure 11 shows the ranges of the spatial geometrical dimensions of the basic unit with a single barb of Figure 9, these dimensions being proportioned with respect to the corresponding dimensions of the basic units according to the USP designation, for the various gauges that can be produced according to the USP specification itself;
Figure 12 is a front perspective view of a basic unit with two barbs;
Figure 13 is a front view of a basic unit with three barbs;
Figure 14 is a schematic illustration of a possible convergent bidirectional suture with two opposed barbs staggered by 180°, with a double change in direction of approximately 90°;
Figures 15, 16, and 17 are perspective views, respectively, of a female-female corner basic unit, a male-male corner basic unit, and a male-female corner basic unit, which can be used for producing angled sutures such as the one shown Figure 13;
Figures 18, 19, and 20 show some examples of convergent unidirectional and bidirectional sutures that can be obtained with the basic units, with one, two, and three barbs, respectively;
Figures 21, 22, 22a, 23, and 23a are side views and perspective views of some examples of unidirectional sutures obtained with basic units with one, two, and three barbs;
Figures 24, 25, and 26 are side views of examples of divergent sutures obtained with basic units with one, two, and three barbs;
Figure 27 is a perspective view of a divergent bidirectional suture obtained with basic units with three barbs;
Figure 28 is a perspective view of a unidirectional suture obtained with basic units with one barb and having a spiral arrangement of the barbs; and
Figure 29 is a perspective view a terminal unit provided with just one connection element.

### DETAILED DESCRIPTION OF THE INVENTION

With reference to the aforesaid figures, as has been mentioned in the foregoing introductory part, the object of the invention is a modular system for providing surgical sutures constituted by a plurality of modular micro-units that can be coupled together, preferably obtained with ultrafine moulding technology, which, once assembled together, give rise to a thread or suture of the desired length in which the barbs are present and distributed on the basis of design specifications.

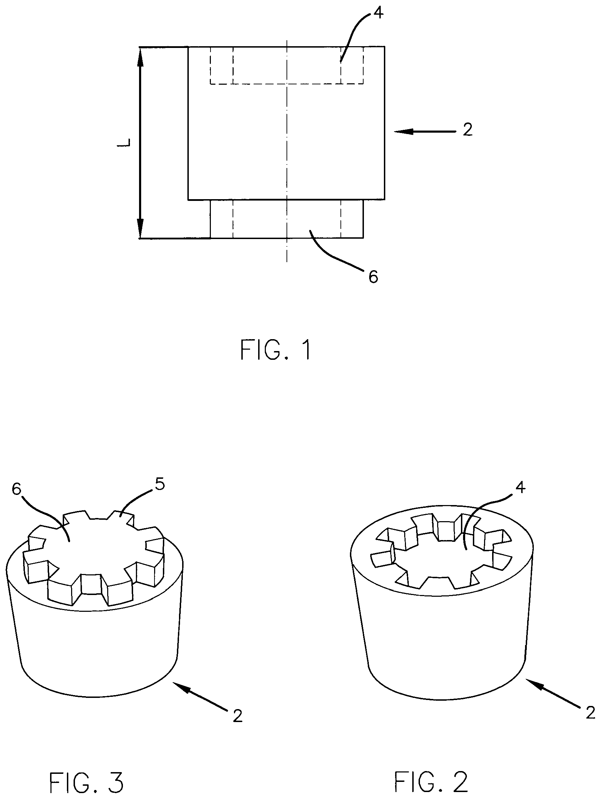

Figure 1 shows by way of example, at an enlarged scale, a basic unit 2 without barbs, which, in a preferred embodiment, is a small cylinder with circular section, provided on the two opposed ends or bases with a male coupling element 4 and a female coupling element 6.

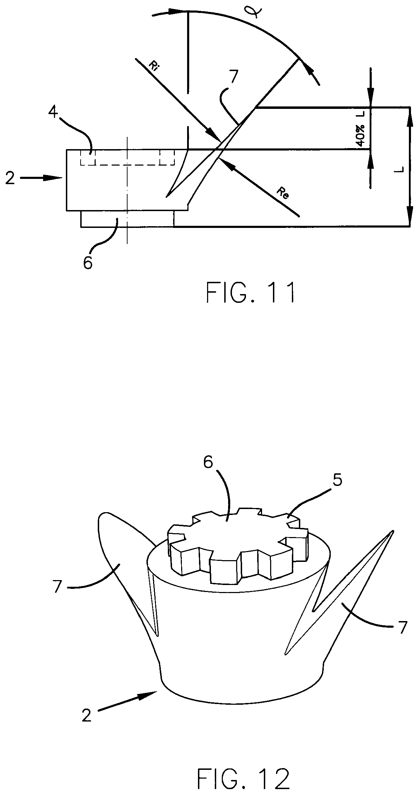

Preferably, but not exclusively, the length L of said basic unit, including the projecting portion of the male coupling means (see Figure 11), is comprised in a range between 0.43 and 2.86 mm.

The coupling elements 4 and 6 are front toothed couplings 5 as shown in Figures 2 and 3, which enable coupling of two micro-units 2 with the freedom of choosing different angular positions about the axis of the units themselves.

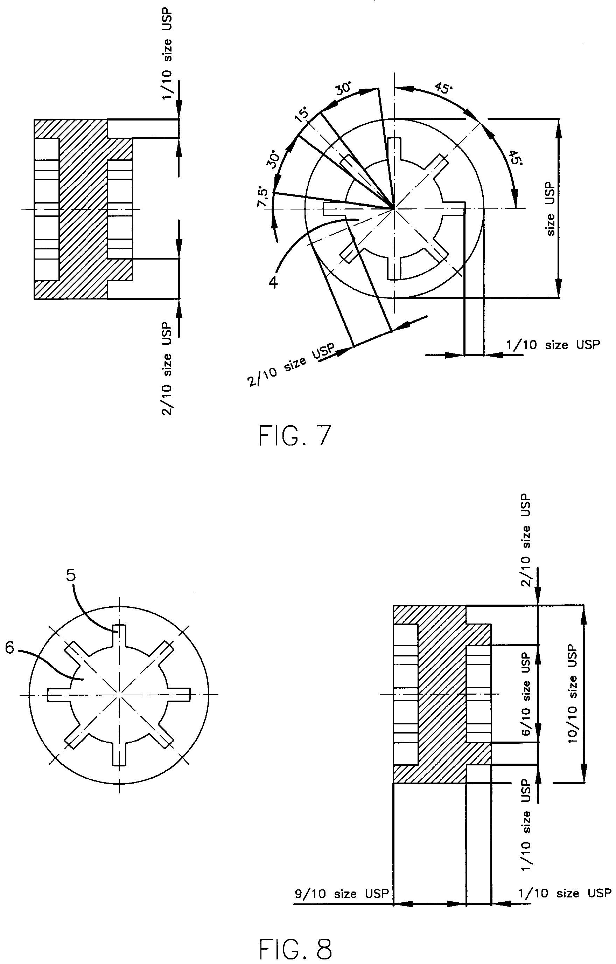

In this connection, as may be seen in Figures 7 and 8, the angular displacement of a tooth 5 of the male coupling 4 of one unit 2 in the female seat 6 of another unit 2 leads to a rotation through 45° of one basic unit with respect to the other. This possibility, which is not important in the case of units without barbs, becomes instead extremely important in the case where on the lateral surface of the basic unit one or more barbs 7 are present variously arranged and oriented, as will be seen hereinafter, in so far as the effects that placing of the thread is able to exert on the tissues of the patient vary. It is known in fact that the divergence of the barbs guarantees lengthening or stretching of the soft tissue in which the thread is placed, whereas their convergence guarantees its compaction or blending, aggregation or protrusion; instead, unidirectional sutures ensure tissue lifting according to preset tensile vectors predetermined by the surgeon.

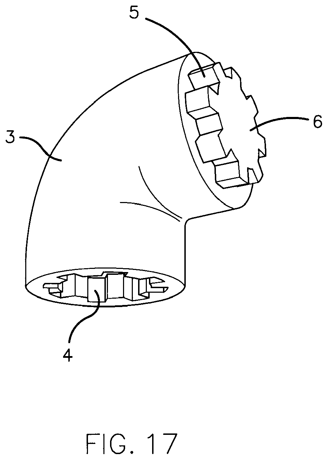

The basic units without barbs 2 can be assembled together in a number equal to N (where N is an integer that may range from 0 to infinity) by coupling the male couplings 4 with the female couplings 6, the sequence of basic units 2 thus obtained giving rise to a non-barbed portion Y of the final suture, as may be seen, for example, in Figures 17, 18, and 19, or else to the entire length of suture.

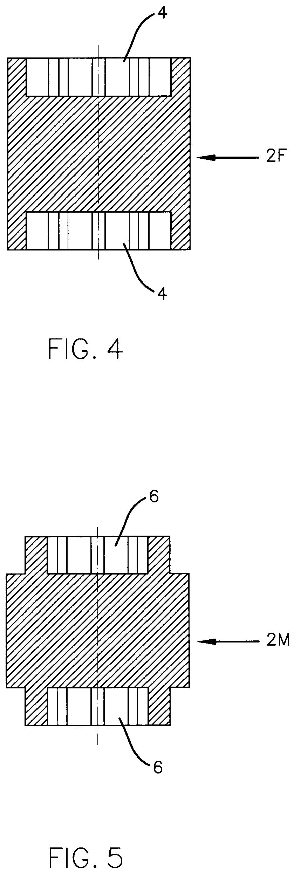

Figures 4 and 5 show the female/female basic unit 2F with two seats 4 at its ends and the male/male basic unit 2M, with two toothed projections 6, which enable, in combination with the male/female basic unit 2, production, with the widest degree of freedom, of both convergent and divergent sutures.

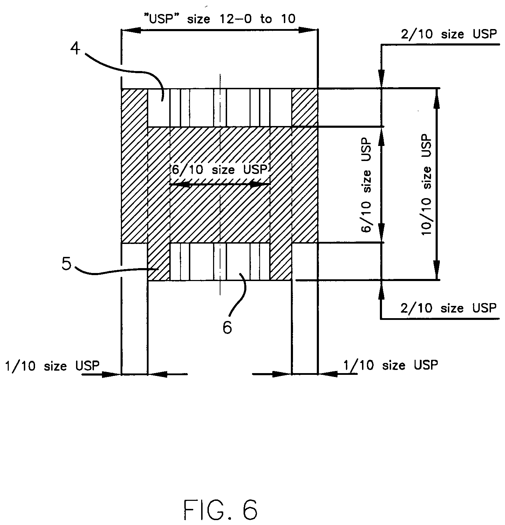

Figures 6, 7, and 8 provide the real parameterized dimensions of the male/female basic unit 2. These dimensions correspond to the USP technical specifications, and may range from a minimum of 0.001 mm to a maximum 1.3 mm of thread diameter.

The ensuing table makes it possible, by choosing a USP reference gauge, to derive the various dimensions of the basic unit on the basis of the central value between the tolerance limits on average diameter envisaged by USP. Thus, for example, if the USP-2 gauge size is chosen, the various dimensions of the basic unit will be derived on the basis of the corresponding central value between the tolerance limits on average diameter envisaged by the USP designation, namely, 0.65 mm.

**USP Table**

| | | Limits on Average Diameter (mm) | | Knot-Pull Tensile Strength (kgf) | | Knot-Pull Tensile Strength (in N) | |
|---|---|---|---|---|---|---|---|
| USP Size | Metric Size (Gauge No.) | Min. | Max. | Limit on Average Min. | Limit on Individual Strand Min. | Limit on Average Min. | Limit on Individual Strand Min. |
| 9-0 | 0.4 | 0.040 | 0.049 | - | - | - | - |
| 8-0 | 0.5 | 0.050 | 0.069 | 0.045 | 0.025 | 0.44 | 0.24 |
| 7-0 | 0.7 | 0.070 | 0.099 | 0.07 | 0.055 | 0.69 | 0.54 |
| 6-0 | 1 | 0.10 | 0.149 | 0.18 | 0.10 | 1.76 | 0.98 |
| 5-0 | 1.5 | 0.15 | 0.199 | 0.38 | 0.20 | 3.73 | 1.96 |
| 4-0 | 2 | 0.20 | 0.249 | 0.77 | 0.40 | 7.55 | 3.92 |
| 3-0 | 3 | 0.30 | 0.339 | 1.25 | 0.68 | 12.2 | 6.67 |
| 2-0 | 3.5 | 0.35 | 0.399 | 2.00 | 1.04 | 19.6 | 10.2 |
| 0 | 4 | 0.40 | 0.499 | 2.77 | 1.45 | 27.2 | 14.2 |
| 1 | 5 | 0.50 | 0.599 | 3.80 | 1.95 | 37.3 | 19.1 |
| 2 | 6 | 0.60 | 0.699 | 4.51 | 2.40 | 44.2 | 25.5 |
| 3 | 7 | 0.70 | 0.799 | 5.90 | 2.99 | 57.8 | 29.3 |
| 4 | 8 | 0.80 | 0.899 | 7.00 | 3.49 | 68.6 | 34.2 |
| | | | | | | | |

| USP Size Min. | | Metric Size (Gauge No.) Max. | | Min. | Max. | | |
|---|---|---|---|---|---|---|---|
| 12-0 | | 0.01 | | 0.001 | 0.009 | - | - |
| 11-0 | | 0.1 | | 0.010 | 0.019 | - | - |
| 10-0 | | 0.2 | | 0.020 | 0.029 | 0.025* | 0.24* |
| 9-0 | | 0.3 | | 0.030 | 0.039 | 0.050* | 0.49* |
| 8-0 | | 0.4 | | 0.040 | 0.049 | 0.07 | 0.69 |
| 7-0 | | 0.5 | | 0.050 | 0.069 | 0.14 | 1.37 |
| 6-0 | | 0.7 | | 0.070 | 0.099 | 0.25 | 2.45 |
| 5-0 | | 1 | | 0.10 | 0.149 | 0.68 | 6.67 |
| 4-0 | | 1.5 | | 0.15 | 0.199 | 0.95 | 9.32 |
| 3-0 | | 2 | | 0.20 | 0.249 | 1.77 | 17.4 |
| 2-0 | | 3 | | 0.30 | 0.339 | 2.68 | 26.3 |
| 0 | | 3.5 | | 0.35 | 0.399 | 3.90 | 38.2 |
| 1 | | 4 | | 0.40 | 0.499 | 5.08 | 49.8 |
| 2 | | 5 | | 0.50 | 0.599 | 6.35 | 62.3 |
| 3 and 4 | | 6 | | 0.60 | 0.699 | 7.29 | 71.5 |
| 5 | | 7 | | 0.70 | 0.799 | - | - |

| | | | | | | | |
|---|---|---|---|---|---|---|---|
| * The tensile strength of the specified USP size is measured by straight pull. | | | | | | | |

It is to be noted that, given the absolute precision with which the individual basic units are produced with ultrafine moulding and the precision with which it is possible to assemble together the basic units using industrial robots, namely, a precision that is of the order of one thousandth of a millimetre, the dimensions of the sutures are all identical to the design dimensions, and hence do not present the variability, along the major axis of the suture, that USP itself contemplates for sutures produced with the extrusion technique.

A peculiar characteristic of the suture obtained according to the present invention is hence that the gauge tolerance is zero: thus, with basic units of the same diameter, the gauge is constant along the major axis of the suture.

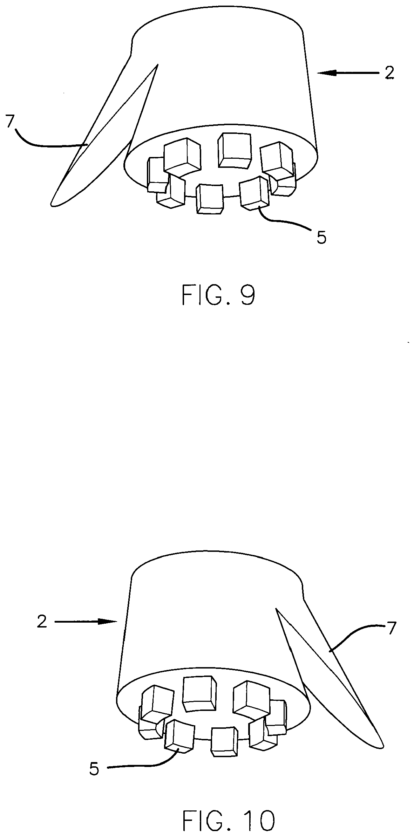

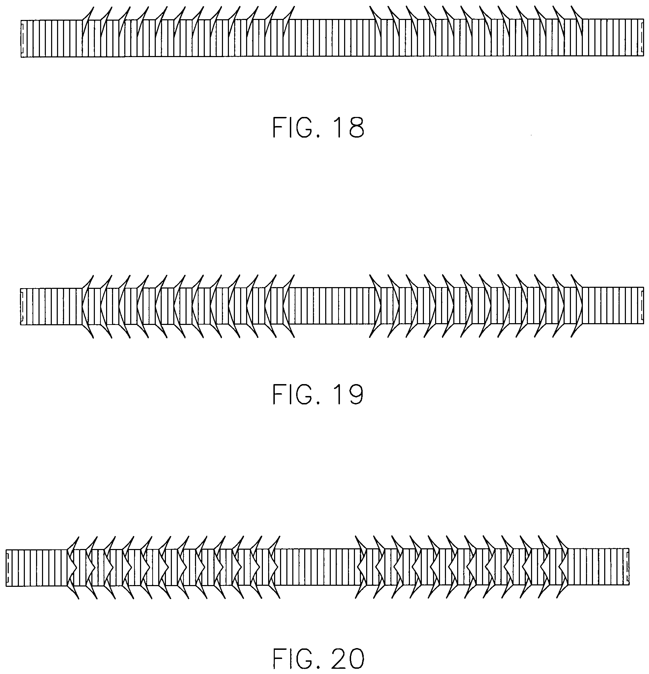

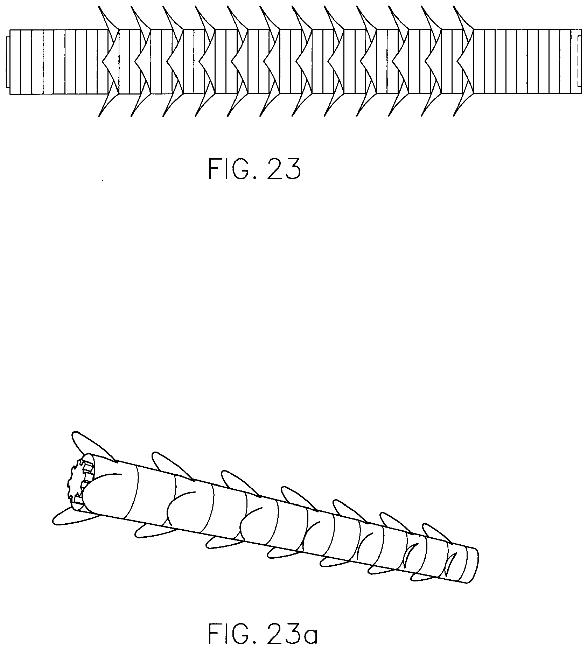

Figures 9 and 10 show two basic units 2 with a single barb 7, which can be assembled together to form a sequence of N units (where N is an integer that may range from 0 to infinity). This sequence of basic units 2 gives rise to a portion, provided with (single) barbs, of the total length of the final suture, which may be either a unidirectional suture or a divergent or convergent or alternating bidirectional suture, or a suture with any sequence whatsoever of the units represented in Figures 20 and 23.

Figure 11 shows the range of spatial geometrical dimensions of the basic unit with a single barb of Figures 9 and 10, with the angle of curvature α of the barb 7 itself, said dimensions being once again proportioned to the corresponding dimensions of the basic units according to the USP designation, for the different gauges that can be produced according to the USP specification referred to above.

For the basic units with one or more barbs, the correct angle of curvature α of the barbs is of extreme importance and is specific for the tissue involved. The range, indicated once again in Figure 11, is comprised between 0° and 90°, with respect to the axis of the abscissae, preferably between 0° and 45°. Vertically the barb 7 extends, with respect to the plane of extension of the female coupling element 4, not beyond 40% of the range comprised between 0.43 mm and 2.86 mm, i.e., of the entire length L of the basic unit. Once again in Figure 11, the internal radius of curvature Ri and external radius of curvature Re of the barb 7 may range from 1 mm to 10 cm.

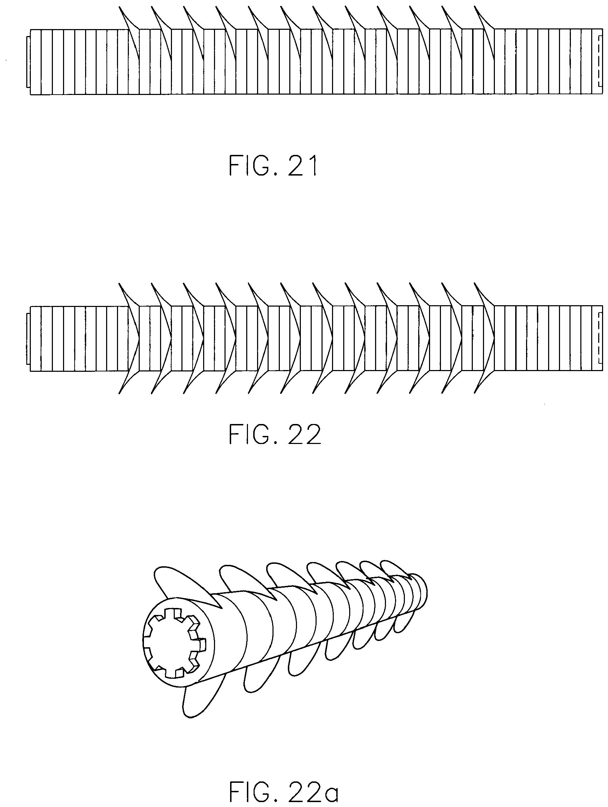

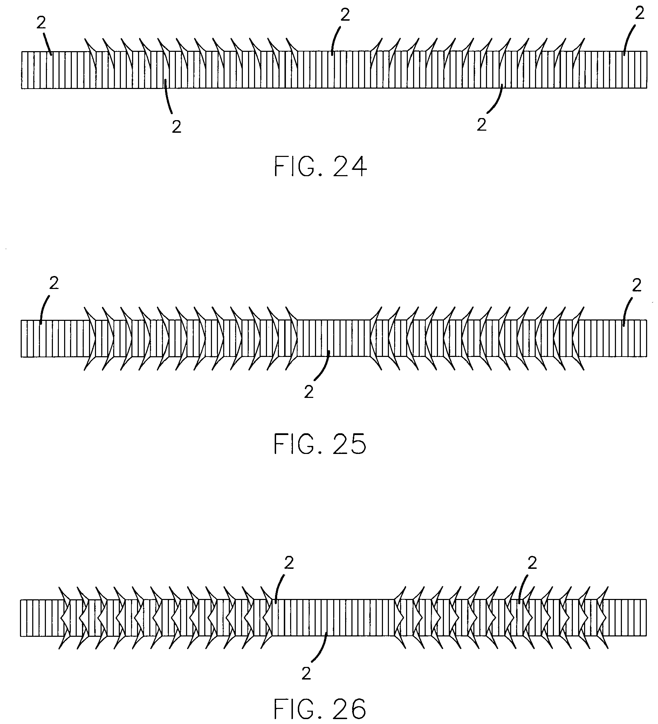

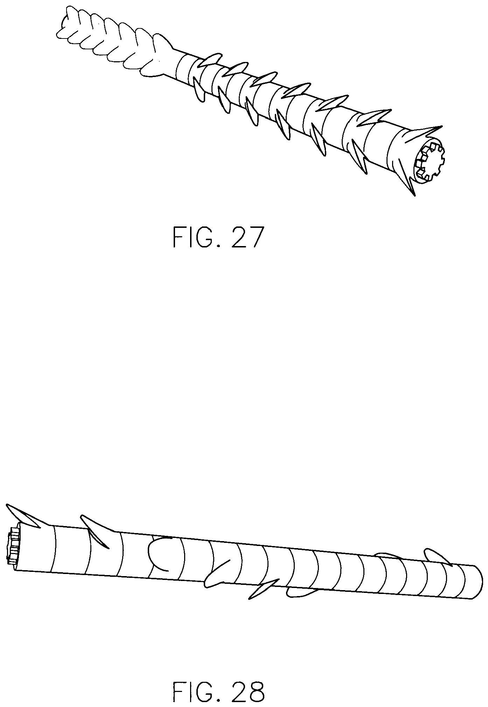

With a basic unit with a single barb it is possible to produce unidirectional sutures as in the examples illustrated in the figures, both with a linear arrangement of the barbs (i.e., with the barbs aligned along the same axis of tangency on the outer margin of the suture as in Figure 21) and with a spiral arrangement (Figure 28), or convergent or divergent bidirectional sutures (Figures 18, 24) or sutures with portions with barbs set opposed two-by-two separated by a small gap without barbs constituted by a number ranging from zero to infinity of the basic units without barbs, or with any other possible arrangement (obtained by slotting the various units available into one another, as with Lego elements).

Figures 9 and 10 show two basic units 2 with a single barb, which, once arranged specular to one another, enable production of sutures with alternately opposed barbs, with a linear or spiral development, either convergent or divergent.

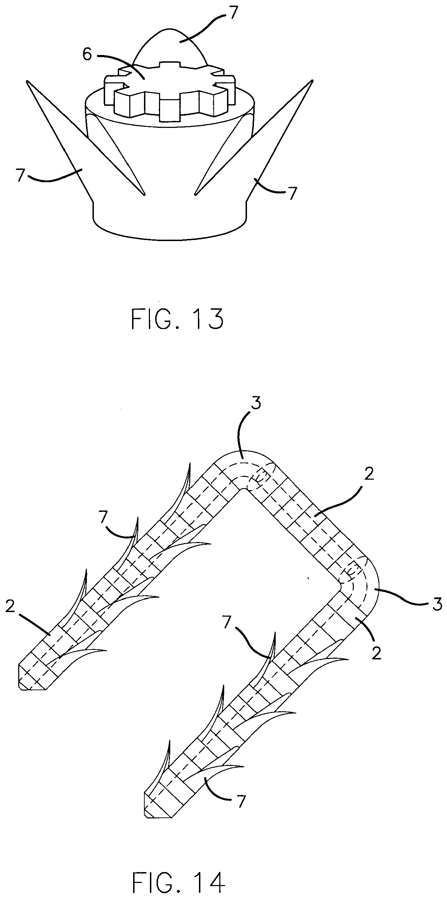

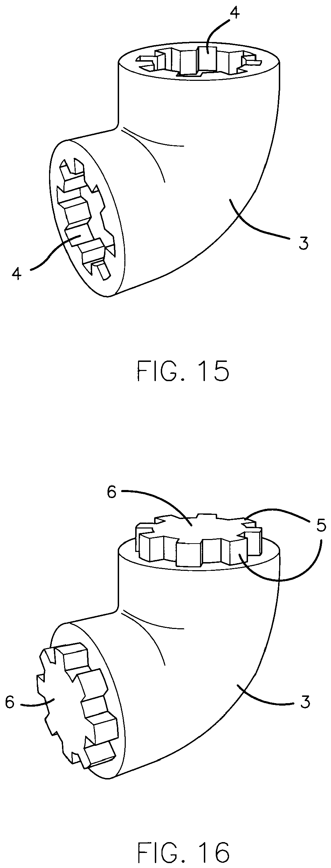

Figure 14 is a schematic representation of a possible convergent bidirectional suture with double barbing opposed and staggered by 180°, with a possible double change in direction of approximately 90°, obtained with modular basic units 2 and with modular corner units 3 (shown in Figures 15, 16, and 17), in which the coupling means are set substantially at 90°.

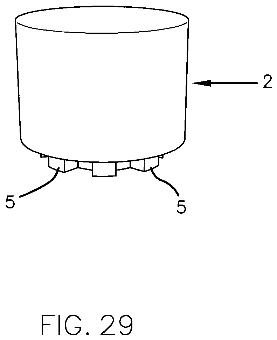

Figure 29 illustrates a terminal unit 2S to be assembled at the end of the suture so as to constitute, if necessary, one of the two ends thereof. It differs from the other basic units in that it has one end without coupling elements.

The object of the disclosure is moreover the method for producing, starting from a plurality of modular micro-units with the characteristics indicated above, a thread or suture of the desired length, which has the mechanical characteristics of a traditional suture and the dimensions of which are proportioned to those of the USP technical specifications.

It has already been said that the modular units that can be coupled together are preferably obtained by ultrafine moulding starting from a thermosensitive material.

It is clear that the individual basic units having the desired characteristics can be obtained also with other industrial technologies, such as for example microtechnologies, which enable products to be obtained having dimensions of the order of microns, or nanotechnologies, which involve manipulation of matter on a subnanometric scale.

Numerous thermosensible materials that may be used. The following may be cited by way of example: esterified hyaluronic acid (EHA), polypropylene (PP), polydioxanone (PDO), caprolactone P(LA-CL), polycaprolactone (PCL), a specific polyester such as polyhydroxyalkanoate (PHA), which is a thermoplastic polymer presenting extraordinary characteristics, polyamides (PAs), PA6, PA66, inert ceramic materials such as alumina (Al₂O₃), or active ceramic materials such as β-whitlockite, and again carbon fibres. It is also possible to resort to metal materials such as platinum and/or titanium.

For production on an industrial scale of the sutures forming the subject of the present patent, once the modular units so far described have been produced, as an alternative to manual assemblage, it is preferable to resort to the use of a robot suitable for industrial production of the sutures forming the subject of the present invention, which will enable the individual basic units to be assembled together according to pre-ordered sequences. Available on the market are robots that are able to recognize, by means of a computerized optical system, the individual basic units and to pick them up with a mechanical arm controlled by the specific software, which enables, after recognition of the specific basic units, assemblage thereof according to the given sequence for the suture being produced and in the correct angular position with respect to the adjacent basic units. In other words, the industrial robot recognizes the basic units, assembles them together according to the specific three-dimensional structure, and then sends the sequence obtained to the subsequent production stages. The suture produced is then put into an oven for welding, where the individual basic units duly coupled together are stably welded to one another following upon a well-defined temperature gradient or with repeated and alternating heating and cooling cycles, for a pre-set time according to the material used for moulding of the basic units.

Said specific material, which is preferably, but not exclusively, a thermoplastic polymer, responds to the specific temperature gradient with a shrinkage in its original volume such as to render impossible disassembly, i.e., detachment of the individual basic units of the suture, since, after the aforementioned thermal treatment, the sequence of basic units behaves as a continuous suture.

## Claims

1. A thread or suture for operations of general and specialized surgery, such as plastic, dermatological, and aesthetic surgery, wherein it is obtained by assembling together modular micro-units of well-defined shape and size, made of thermosensitive material, which are equipped with co-operating connection means that enable coupling of said micro-units to one another in the production stage, to obtain either smooth sutures or sutures provided with protuberances or barbs or protrusions, or sutures of different shapes, or combinations thereof, according to the various multiple ways in which the specific modular units can be assembled or slotted together to achieve the desired length, **characterised in that**:
said co-operating connection means are constituted by male and female front-coupling means,
said male and female front-coupling means are toothed coupling means, angular displacement of a tooth of the male coupling relative to the female coupling at each connection leading to rotation of one modular unit with respect to the other, so as to enable production of sutures in which the barbs or protuberances, if present, may be distributed along the outer surface of the suture on the basis of design specifications.

2. The thread or suture as per Claim 1, **characterized in that** the modular units may be provided or not, on their own lateral surface, with one or more barbs variously arranged and oriented, allowing unidirectional suture, with both a linear and spiral arrangement, and/or convergent or divergent bidirectional sutures, with said barb being **characterized in that**:
- the angle of curvature of the barbs (α) ranged from 0° to 90°, preferably from 0° to 45°,
- vertically the barb extends with respect to the plane of extension of the female coupling element, not beyond the 40% of the range comprised between 0.43 mm and 2.86 mm,
- the internal radius of curvature (Ri) and external radius of curvature (Re) ranged from 1 mm to 10 cm.

3. The thread or suture as per any one of the preceding claims, **characterized in that** the real parameterized dimensions of the male/female (2), male/male (2M), and female/female (2F) basic units correspond to the technical specifications of the United States Pharmacopoeia (USP), and namely, with a thread gauge ranging from a minimum of 0.001 mm to a maximum of 1.3 mm.

4. The thread or suture as per any one of the preceding claims, **characterized in that** the modular unit has a length (L) comprised between 0.30 mm and 3.20 mm, preferably between 0.50 mm and 3.00 mm, even more preferably between 0.43 mm and 2.86 mm.

5. The thread or suture as per any one of the preceding claims, **characterized in that** the angles (α) of the barbs are comprised between 0° and 90° where at 0° the barb is understood as being parallel to the axis of the thread.

## Patentansprüche

1. Faden oder Naht für Operationen allgemeiner und spezialisierter Chirurgie wie plastischer, dermatologischer und ästhetischer Chirurgie, wobei dieser (diese) durch Zusammensetzen von modularen Mikroeinheiten mit gut definierter Form und Größe aus wärmeempfindlichem Material bestehen, die mit zusammenwirkenden Verbindungsmitteln ausgestattet sind, die das Aneinanderkoppeln der Mikroeinheiten auf der Produktionsstufe ermöglichen, um entweder glatte Nähte zu erhalten oder Nähte, die mit Protuberanzen oder Stacheln oder Ausbuchtungen versehen sind, oder Nähte von verschiedenen Formen oder Kombinationen davon entsprechend den verschiedenen zahlreichen Arten und Weisen, auf denen die spezifischen modularen Einheiten zusammengesetzt oder ineinander gesteckt werden können, um die gewünschte Länge zu erhalten, **dadurch gekennzeichnet, dass**:
die zusammenwirkenden Verbindungsmittel aus männlichen und weiblichen Frontkoppelmitteln bestehen,
die männlichen und weiblichen Frontkoppelmittel gezahnte Koppelmittel sind, wobei eine Winkelverschiebung eines Zahns der männlichen Kopplung in Bezug auf die weibliche Kopplung an jeder Verbindung zu Rotation einer modularen Einheit in Bezug auf die andere führt, um die Herstellung von Nähten zu ermöglichen, bei denen die Stacheln oder Protuberanzen, falls vorhanden, entlang der Außenseite der Naht auf der Basis von Entwurfsspezifikationen verteilt sein können.

2. Faden oder Naht nach Anspruch 1, **dadurch gekennzeichnet, dass** die modularen Einheiten auf ihrer eigenen Seitenfläche mit einem oder mehreren, unterschiedlich angeordneten und ausgerichteten Stacheln versehen sein können oder nicht, die eine unidirektionale Naht mit sowohl einer linearen als auch spiraligen Anordnung zulassen, und/oder konvergierende oder divergierende bidirektionale Nähte zulassen, wobei der Stachel **dadurch gekennzeichnet ist, dass**:
- der Krümmungswinkel der Stacheln (α) von 0° bis 90°, vorzugsweise von 0° bis 45°, reichte,
- vertikal der Stachel in Bezug auf die Ausdehnungsebene des weiblichen Kopplungselements sich nicht über 40 % des zwischen 0,43 mm und 2,86 mm liegenden Bereichs erstreckt,
- der innere Krümmungsradius (Ri) und äußere Krümmungsradius (Re) von 1 mm bis 10 cm reichten.

3. Faden oder Naht nach einem der vorstehenden Ansprüche, **dadurch gekennzeichnet, dass** die realen, parametrisierten Abmessungen der männlichen/weiblichen (2), männlichen/männlichen (2M) und weiblichen/weiblichen (2F) Grundeinheiten den technischen Spezifikationen der United States Pharmacopoeia (USP) entsprechen und zwar mit einem Fadenmaß, das von einem Minimum von 0,001 mm bis zu einem Maximum von 1,3 mm reicht.

4. Faden oder Naht nach einem der vorstehenden Ansprüche, **dadurch gekennzeichnet, dass** die molekulare Einheit eine Länge (L) aufweist, die zwischen 0,30 mm und 3,20 mm, vorzugsweise zwischen 0,50 mm und 3,00 mm, noch mehr bevorzugt zwischen 0,43 mm und 2,86 mm liegt.

5. Faden oder Naht nach einem der vorstehenden Ansprüche, **dadurch gekennzeichnet, dass** die Winkeln (α) der Stacheln zwischen 0° und 90° liegen, wobei der Stachel bei 0° als parallel zur Achse des Fadens verstanden wird.

## Revendications

1. Fil ou suture pour des opérations chirurgicales à caractère général et spécialisées telles que la chirurgie plastique, dermatologique et esthétique, dans lequel il est obtenu par assemblage de micro-unités modulaires de forme et de taille bien définies, constituées d'un matériau thermosensible, qui sont pourvues de moyens de raccordement coopératifs qui permettent le couplage desdites micro-unités les unes aux autres lors de l'étape de production, pour obtenir soit des sutures lisses, soit des sutures portant des protubérances ou des barbes ou des saillies, ou des sutures de différentes formes, ou des combinaisons de celles-ci, selon les multiples différentes manières dont les unités modulaires spécifiques peuvent être assemblées ou emboîtées par l'intermédiaire d'encoches pour obtenir la longueur souhaitée, **caractérisé en ce que** :
lesdits moyens de raccordement coopératifs sont constitués de moyens de couplage avant mâle et femelle,
lesdits moyens de couplage avant mâle et femelle sont des moyens de couplage à dents, le déplacement angulaire d'une dent du moyen de couplage mâle par rapport au moyen de couplage femelle au niveau de chaque raccord conduisant à la rotation d'une unité modulaire par rapport à l'autre, de manière à permettre la production de sutures dans lesquelles les barbes ou protubérances, le cas échéant, peuvent être distribuées le long de la surface extérieure de la suture sur la base des spécifications de conception.

2. Fil ou suture selon la revendication 1, **caractérisé en ce que** les unités modulaires peuvent être dotées ou non, sur leur propre surface latérale, d'une ou plusieurs barbes différemment agencées et orientées, permettant la suture unidirectionnelle, avec un agencement linéaire et en spirale, et/ou des sutures bidirectionnelles convergentes ou divergentes, lesdites barbes étant **caractérisées en ce que** :
- l'angle de courbure des barbes (α) se trouve dans la plage de 0° à 90°, de préférence de 0° à 45°,
- verticalement la barbe s'étend par rapport au plan d'extension de l'élément de couplage femelle, pas au-delà de 40 % de la plage comprise entre 0,43 mm et 2,86 mm,
- le rayon interne de courbure (Ri) et le rayon externe de courbure (Re) se trouvent dans la plage de 1 mm à 10 cm.

3. Fil ou suture selon l'une quelconque des revendications précédentes **caractérisé en ce que** les dimensions paramétrées des unités basiques mâle/femelle (2), mâle/mâle (2M), et femelle/femelle (2F) correspondent aux spécifications techniques de la Pharmacopée américaine (USP), et précisément, avec un calibre de fil dans la plage d'un minimum de 0,001 mm à un maximum de 1,3 mm.

4. Fil ou suture selon l'une quelconque des revendications précédentes, **caractérisé en ce que** l'unité modulaire a une longueur (L) comprise entre 0,30 mm et 3,20 mm, de préférence entre 0,50 mm et 3,00 mm, de manière encore davantage préférée entre 0,43 mm et 2,86 mm.

5. Fil ou suture selon l'une quelconque des revendications précédentes, **caractérisé en ce que** les angles (α) des barbes sont compris entre 0° et 90° où à 0° la barbe est comprise comme étant parallèle à l'axe du fil.
